(19) Europäisches Patentamt
European Patent Office
Office européen des brevets

(11) EP 0 489 655 B1

(12) **FASCICULE DE BREVET EUROPEEN**

(45) Date de publication et mention de la délivrance du brevet: **28.05.1997 Bulletin 1997/22**

(51) Int. Cl.[6]: **C07D 498/20**, G03C 1/685 // (C07D498/20, 265:00, 249:00, 209:00)

(21) Numéro de dépôt: **91403280.0**

(22) Date de dépôt: **04.12.1991**

(54) **Composés photochromiques de type indolino-spiro-benzoxazine et leur procédé de préparation**

Photochrome Indolinospirobenzoxazine und Verfahren zu ihrer Herstellung

Indolino-spiro-benzoxazine photochromic compounds and process for their preparation

(84) Etats contractants désignés: **CH DE ES GB IT LI**

(30) Priorité: **06.12.1990 FR 9015324**

(43) Date de publication de la demande: **10.06.1992 Bulletin 1992/24**

(73) Titulaire: **ESSILOR INTERNATIONAL (Compagnie Générale d'Optique)**
**F-94220 Charenton-le-Pont (FR)**

(72) Inventeurs:
- **Guglielmetti, Robert**
  **F-13009 Marseille (FR)**
- **Tardieu, Pascale**
  **F-13004 Marseille (FR)**

(74) Mandataire: **Thibon-Littaye, Annick**
**Cabinet A. THIBON-LITTAYE**
**11 rue de l'Etang**
**78160 Marly-le-Roi (FR)**

(56) Documents cités:
**EP-A- 0 245 020**
**EP-A- 0 339 661**
**EP-A- 0 358 774**
**WO-A-87/00524**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.6/3.4

## Description

La présente invention a pour objet des composés qui sont du type indolino-spiro-oxazine et qui présentent des propriétés photochromiques utiles. Elle concerne également un procédé de préparation de ces composés, ainsi que leurs applications industrielles en tant qu'additifs photochromiques, notamment dans la fabrication d'articles transparents photochromiques.

On connait déjà divers composés photochromiques appartenant à la famille chimique des spiro-oxazines, y compris parmi ceux qui présentent la formule d'une indolino-spiro-benzoxazine, soit :

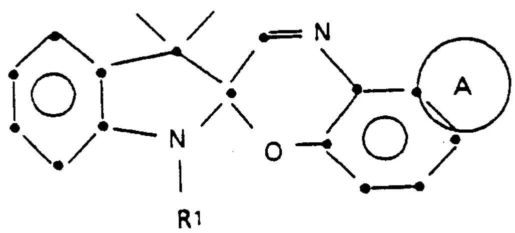

(I)

où $R^1$ est un radical alkyle et les noyaux indoline et oxazine peuvent porter des substituants variés, notamment des radicaux alkyle inférieur (comportant de 1 à 5 atomes de carbone) qui ne perturbent pas leurs propriétés essentielles.

Cependant, il reste un grand besoin de trouver des perfectionnements aux techniques de fabrication des composés photochromiques et de découvrir des composés nouveaux, dans lesquels on recherche des propriétés spécifiques, différentes de celles des composés déjà connus.

Si l'on considère en particulier le domaine de la fabrication des verres ou lentilles organiques, propres à se colorer en lumière solaire, il est intéressant de disposer d'une vaste gamme d'additifs photochromiques, différant les uns des autres par exemple par leurs propriétés de compatibilité avec telle ou telle résine organique, par le spectre d'absorption de la forme colorée, etc., tout en respectant au mieux d'autres impératifs tels que la rapidité de coloration et décoloration, la stabilité chimique, la résistance à des conditions d'emploi impliquant dans le temps un grand nombre de séquences de coloration et décoloration et l'insensibilité à des variations de température.

Les composés suivant l'invention sont des indolinospiro-benzocyclodiazènes, c'est-à-dire des composés répondant à la formule I dans laquelle le groupe A comporte un cycle à liaison diazène -N=N-, de préférence dans un cycle à cinq chaînons triaza orthocondensé sur un cycle aromatique. Ils se distinguent en cela d'autres composés proposés dans l'art antérieur, en particulier de ceux où le groupement A, orthocondensé sur le noyau benzénique de la partie oxazine de la molécule, est un hétérocycle azoté aromatique parmi ceux divulgués par le brevet américain US 4 720 547, ou de ceux où ce groupement A est de type benzofurane comme dans la demande de brevet européen 0 358 774.

D'autres composés photochromiques de type spirooxazine sont cités dans l'art antérieur avec des substituants tels que des chaînes aromatiques ou alicycliques avec un atome d'azote dans la demande de brevet européen EP-A-0 245 020, avec un groupe benzyl comme substituant dans la demande de brevet européen EP-A-0 339 661, avec un ou deux noyaux hétéroaromatiques à un ou deux azotes dans la demande de brevet WO-A-87/00524.

Les composés photochromiques de l'invention comportent en groupement A un cycle triazolo. Ils présentent la la formule d'un indolinospiro-benzo-cyclodiazène à groupement benzotriazole, la partie oxazinique de la molécule comportant un cycle triaza orthocondensé sur un cycle aromatique, lesdits composés présentant la formule développée (II) :

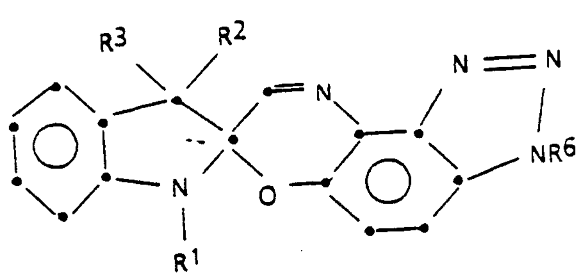

(II)

dans laquelle :

- $R^6$ est un atome d'hydrogène, un phényle, un radical alkyle,
- $R^1$ représente :

  i) un groupe alkyle de 1 à 8 atomes de carbone,
  ii) un groupe allyle, phényle, arylalkyle,
  iii) un groupe alicyclique,
  iv) un groupe hydrocarbure aliphatique comportant dans sa chaîne un ou plusieurs hétéroatomes O, N ou S, une fonction acide, ester ou alcool ;

- $R^2$ et $R^3$ peuvent chacun et indépendamment l'un de l'autre représenter un groupe $C_{1-8}$ alkyle, phényle, phényle mono et disubstitué par des groupes $C_{1-4}$ alkyle et/ou $C_{1-5}$ alkoxy ou peuvent être combinés pour former une chaîne cyclique de 6 à 8 atomes de carbone (incluant le carbone spirannique 3 de l'hétérocycle indolinique) ;

  et en ce qu'ils comportent de 1 à 4 substituants $R^4$ sur le noyau phényle de la partie indolinique de la molécule et/ou 1 ou 2 substituants $R^5$ sur le noyau phényle de la partie oxazinique, $R^4$ et $R^5$ pouvant chacun et indépendamment l'un de l'autre représenter :

  i) un atome d'hydrogène, une fonction amine NR'R", où R' et R" représentent chacun et indépendamment un atome d'hydrogène, un groupe alkyle, cycloalkyle, phényl ; R' et R" peuvent se combiner pour former un cycloalkyle et contenir un ou plusieurs hétéroatomes ;
  ii) un groupe R, OR, SR, COR ou COOR dans lequel R représente un atome d'hydrogène, un groupe alkyle de 1 à 6 atomes de carbone ou un groupe aryle ou hétéroaryle ;
  iii) un atome d'halogène, un groupe $C_{1-4}$ monohaloalkyle, un groupe $C_{1-4}$ polyhaloalkyle,
  iv) $-NO_2$, CN, SCN.

Chacun des substituants $R^4$ peuvent être présent sur l'un quelconque des atomes de carbone convenables de la partie indoline du composé photochromique, en position 4, 5, 6 et 7.

Il est toutefois préférable que deux substituants autres que H soient présents en positions 4 et 5, 6 et 7, 4 et 6 ou 6 et 7.

Des composés préférés de l'invention répondent à l'une ou l'autre des caractéristiques suivantes :

- $R^6$ est un atome d'hydrogène avec un radical alkyle comportant de 1 à 5 atomes de carbone,
- $R^1$ est un groupe méthyle, éthyle, n-propyle, isopropyle ou n-butyle, et de préférence un groupe méthyle ou isopropyle,
- ou $R^1$ est un groupe benzyle, phényle mono et disubstitué par des substituants du type alkyle ou alkoxy de 1 à 6 atomes de carbone,
- $R^2$ et $R^3$ peuvent chacun et indépendamment l'un de l'autre représenter un groupe méthyle ou éthyle, et de préférence un groupe méthyle.

De préférence :

- $R^1$ est un groupe $C_{1-4}$ alkyle, phényle ou benzyle ;
- $R^2$ et $R^3$ sont choisis parmi les groupes $C_{1-5}$ alkyle tels que méthyle et éthyle ou un groupe phényle, ou sont combinés pour former un groupe cyclohexyle ;
- chacun des groupes $R^4$ est choisi parmi le groupe constitué par hydrogène, $C_{1-2}$ alkyle, chlore, fluor, brome, iode, $C_{1-2}$ trihaloalkyle et $C_{1-5}$ alkoxy ;
- et $R^5$ est un atome d'hydrogène ou un groupe $C_{1-4}$ alkoxy ou une amine tertiaire ou un halogène.

Parmi les composés selon l'invention sont particulièrement intéressants les composés dans lesquels :

- $R^1$ est un groupe $C_{1-4}$ alkyle tel que méthyle, éthyle, isopropyle ou n-butyle ;
- $R^2$ et $R^3$ représentent chacun et indépendamment l'un de l'autre un groupe méthyle, éthyle ou phényle ;
- $R^4$ est un atome d'hydrogène, un groupe méthyle, méthoxy ou chloro ;
- et $R^5$ est un atome d'hydrogène.

Parmi d'autres applications privilégiées des composés de l'invention, il convient de citer spécialement l'emploi des additifs photochromiques dans la fabrication de disques d'enregistrement d'informations optiques tels que ceux que l'on appelle couramment disques laser.

Dans ce cas, il est souhaitable que le cycle diazène de la molécule comporte un proton, c'est-à-dire notamment que $R^6$ soit un atome d'hydrogène dans la formule II.

Par ailleurs, on a pu constater que l'on a souvent avantage à utiliser des composés dans lesquels la partie indolinique de la molécule comporte un substituant nitro, de préférence en position 5 du groupement indoline. En particulier la colorabilité photochromique est sensiblement augmentée lorsque dans la formule II, le substituant $R^4$ est $NO_2$ de préférence en position 5.

Les composés de l'invention peuvent être obtenus par un procédé de préparation exploitant des méthodes de synthèse en elles-même connues, qui comporte une étape de condensation d'une base indolinique III sur un composé nitroso-hydroxy-hétéroaromatique IV, suivant le schéma :

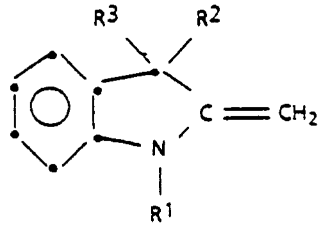

(III)

+

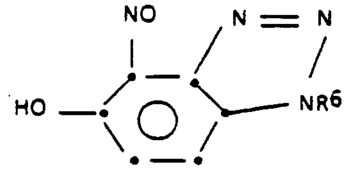

(IV)

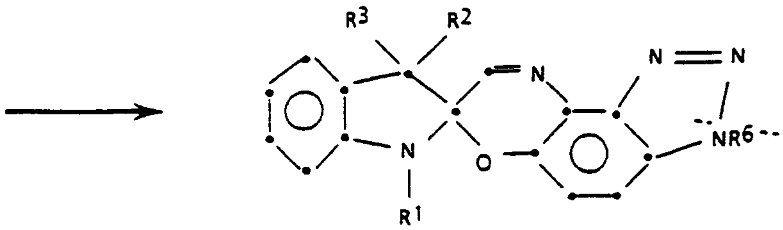

(II)

Le composé intermédiaire (IV) peut être préalablement obtenu par hydrolyse d'un dérivé approprié que l'on convertit ainsi en dérivé hydroxy correspondant, puis nitrosation de ce dernier. Le dérivé hydroxy convenable peut lui-même être obtenu à partir du para-aminophénol par des étapes successives d'acétylation, nitration, réduction catalytique transformant $NO_2$ en $NH_2$, et condensation pour former le cycle triazolique. L'acétoxy-5 acétyl-1 benzotriazole obtenu à ce stade peut être désacétylé par hydrolyse en milieu d'acide fort pour conduire à l'hydroxy-5 benzotriazole recherché pour nitrosation.

Dans leur mise en oeuvre pour la préparation de compositions photochromiques, les composés selon la présente invention peuvent être dissous dans un solvant convenable tel que le toluène ou l'éthanol afin d'obtenir une solution photochromique. Ces mêmes composés photochromiques peuvent également être dissous au sein d'un polymère, d'un copolymère ou d'un mélange de polymères en solution dans un solvant organique convenable.

Ils entrent ainsi dans la constitution de compositions selon la présente invention, qui peuvent être appliquées ou introduites dans ou sur un matériau polymère organique transparent pour obtenir un article transparent photochromique. Préférentiellement, ce matériau est un matériau de qualité optique, plus particulièrement un matériau convenable pour la fabrication de lentilles ophtalmiques.

Ils peuvent aussi entrer dans des compositions photochromiques faisant également l'objet de la présente invention qui peuvent être utilisées directement dans la constitution de films plastiques photochromiques, de plaques et de lentilles telles que des lentilles pour lunettes de soleil, viseurs, optiques de caméra et filtres.

Des exemples de compositions appropriées, conformément à l'invention, pour la fabrication de matériaux et articles transparents photochromiques, comportent un ou plusieurs des composés photochromiques de la présente invention en combinaison avec un ou plusieurs des polymères suivants :
polymère d'un monomère de polyolallylcarbonate, poly-acrylate, polyalkyl-acrylates tels que le polyméthacrylate de méthyle (PMMA), acétate de cellulose, triacétate de cellulose, propiono et butyro-acétate de cellulose, acétate de polyvinyle, alcool polyvinylique, polyuréthanes, polycarbonates, polyéthylènetéréphtalate, polystyrène, copolymères de styrène et de méthacrylate de méthyle, acrylonitrile, polyvinylbutyral.

La quantité de composé photochromique (ou de composition contenant ce composé) appliquée ou introduite sur ou dans le matériau polymère n'est pas d'importance critique et elle dépend généralement de l'intensité de couleur désirée sous irradiation et de la méthode utilisée pour incorporer ou appliquer le composé photochromique. Cette dernière peut être choisie parmi les nombreuses méthodes applicables aux composés photochromiques de l'art antérieur, parmi lesquelles notamment la dissolution ou la dispersion du composé dans la composition de base du matériau, ou la réalisation d'une couche photochromique en surface ou à l'intérieur d'un matériau support transparent.

D'une manière générale, plus on ajoute de composé photochromique, plus la coloration sous irradiation sera importante. Une telle quantité peut être décrite comme une quantité photochromique. De façon usuelle, la quantité de composé photochromique incorporée au matériau optique est de 0,01 à 20 % en poids, et préférentiellement de 0,05 à 10 % en poids, par rapport au poids total de matériau optique.

On obtient ainsi des effets photochromiques qui se traduisent par l'apparition d'une coloration sous exposition à des radiations appartenant au domaine de l'U.V., avec retour à la couleur ou la transparence originelle lorsqu'on interrompt l'exposition aux radiations U.V. Ce changement de coloration peut se renouveler un très grand nombre de fois, comme il est demandé pour des lunettes de protection solaire. De plus, la coloration persiste pendant tout le temps de l'exposition au rayonnement solaire mieux que dans le cas des composés photochromiques de l'art antérieur.

L'invention sera maintenant plus complètement illustrée au moyen d'exemples particuliers de mise en oeuvre non limitatifs.

**EXEMPLE I**

SYNTHESE DU TRIMETHYL-INDOLINO-SPIROBENZOTRIAZOLE-OXAZINE

1) Synthèse de l'hydroxy-5 nitroso-4 benzotriazole

Acétoxy-4 acétylamino-1 benzène

A une solution agitée de 0,05 mole de p-aminophénol dans 50 ml de NaOH (3M), on ajoute rapidement 15 ml d'anhydride acétique et 5 à 10 g de glace pilée. Une agitation vigoureuse est maintenue pendant quelque minutes. Un précipité beige apparaît qui est recueilli et purifié si nécessaire par chromatographie (éluant $CHCl_3/CH_3OH$).

| | |
|---|---|
| Rendement | 80 % |
| Point de fusion | 152 °C |
| Poids moléculaire | 193 g |
| Formule brute | $C_{10}H_{11}NO_3$ |

L'analyse en résonance magnétique nucléaire et spectrométrie dans l'infrarouge révèle la formule développée de l'acétoxy-4 acétyl amino-1 benzène.

Acétoxy-4 acétylamino-1 nitro-2 benzène

1,93 g de l'acétoxy-4 acétylamino-1 benzène obtenu (0,01 mole) sont ajoutés par petites portions sur 2 ml d'acide nitrique fumant ,agités à basse température (bain de glace + sel, T : 0 - 5 °C).

Durant 20-25 mn, le mélange réactionnel est ensuite maintenu à une température comprise entre 12 et 15 °C, puis versé sur de la glace pilée (hydrolyse).

Le solide jaune vif qui précipite est recueilli et recristallisé dans l'éthanol. Il est analysé par résonance magnétique nucléaire et par spectrométrie infrarouge en solution dans le chloroforme.

| | |
|---|---|
| Rendement avant recristallisation | 97 % |
| Point de fusion | 142 °C |
| Poids moléculaire | 238 g |
| Rendement après recristallisation | 68 % |
| Formule brute | $C_{10}H_{10}N_2O_5$ |
| Point de fusion | 144 °C |

Acétoxy-4 acétylamino-1 amino-2 benzène

Un mélange comprenant 2,38 g de l'acétoxy-4 acétylamino-1 nitro-2 benzène obtenu (0,01 mole), 6 ml de cyclohexène, du palladium sur charbon 10 % (en quantité catalytique) dans 20 ml d'éthanol, est porté à reflux sous atmosphère d'hydrogène durant une nuit.

Le lendemain, on filtre à chaud sur célite afin d'éliminer le catalyseur. Au fur et à mesure que le filtrat refroidit, on observe l'apparition d'un précipité blanc qui est recueilli et purifié par recristallisation dans l'éthanol absolu. L'analyse par résonance magnétique nucléaire et infrarouge montre qu'il s'agit d'acétoxy-4 acétylamino-1 amino-2 benzène.

| | |
|---|---|
| Rendement | 64 % |
| Point de fusion | 174 °C |
| Poids moléculaire | 208 g |
| Formule brute | $C_{10}H_{12}N_2O_3$ |

Acétoxy-5 acétyl-1 benzotriazole

A une solution de 2,08 g d'acétoxy-4 acétylamino-1 amino-2 benzène (0,01 mole), 1,9 ml d'acide chlorhydrique concentré, 10 ml d'eau distillée, maintenue à 0 °C, on ajoute très lentement une solution de 0,7 g de nitrite de sodium $NaNO_2$ (0,01 mole) dans 2,7 ml d'eau distillée.

Un précipité orange se forme au fur et à mesure de l'addition. Il est recueilli, filtré et lavé à l'eau.

| | |
|---|---|
| Rendement | 94 % |
| Point de fusion | 125 °C |
| Poids moléculaire | 219 g |
| Formule brute | $C_{10}H_9N_3O_3$ |

Chlorhydrate d'hydroxy-5 benzotriazole

On porte à reflux, jusqu'à complète solubilisation, un mélange de 2,19 g (0,01 mole) d'acétoxy-5 acétylbenzotriazole dans 7 ml d'acide chlorhydrique concentré.

La solution est ensuite refroidie à basse température (0 °C). Un précipité se forme qui est recueilli et lavé à l'eau distillée.

| | |
|---|---|
| Rendement | 65 % |
| Point de fusion | 220 °C |
| (F littérature | 225 °C) |
| Poids moléculaire | 171,5 g |
| Formule brute | $C_6H_5N_3O$ |

Hydroxy-5 nitroso-4 benzotriazole

A une solution de 1,72 g (0,001 mole) de chlorhydrate d'hydroxy-5 benzotriazole dans 5 ml d'eau distillée, maintenue à 0 °C par un bain de glace sur sel, on ajoute en 15 mn un mélange de 0,69 g de $NaNO_2$ (0,001 mole) dans 5 ml

d'eau distillée.

L'addition terminée, on poursuit l'agitation à basse température durant 1 heure.

Le solide jaune orangé qui s'est formé est ensuite filtré puis séché.

| | |
|---|---|
| Rendement | 95 % |
| Poids moléculaire | 164 g |
| Formule brute | $C_6H_4N_4O_2$ |

2) Synthèse du trimethyl-indolino-spirobenzotriazolooxazine

On porte à reflux 0,003 mole de la base indolinique de formule triméthyl-1,3,3 méthylène-2 indoline, dans 20 ml d'un solvant, constitué ici d'éthanol absolu. On ajoute lentement 0,003 mole d'hydroxy-5 nitroso-4 benzotriazole en suspension dans 60 ml de solvant (addition en 1 h 30). Le reflux est poursuivi 2 h 30.

Au fur et à mesure de l'avancement de la réaction l'eau est éliminée avec le solvant par formation d'un azéotrope (montage avec un équipement Dean-Stark). On récupère en fin de réaction du produit nitrosé de départ n'ayant pas réagi, dans la proportion de 22 % en poids.

Le produit obtenu est purifié par chromatographie sur silice dans un éluant constitué d'un mélange éther/acétate d'éthyle. Il répond à la formule développée II avec $R^1 = R^2 = R^3$ étant tous trois - $CH_3$.

| | |
|---|---|
| Poids moléculaire | 319 g |
| Formule brute | $C_{18}H_{17}N_5O$ |

**EXEMPLE II :**

Synthèse de l'isopropyl-diméthyl-indolino-spirobenzotriazole-oxazine

On applique la même procédure que dans l'exemple II pour provoquer la condensation du produit de l'exemple I avec la base indolinique isopropyl-1 méthylène-2 diméthyl-3,3 indoline.

On obtient ainsi l'homologue isopropyldiméthyle présentant la formule II avec $R^1$ étant le radical isopropyle et $R^2$ et $R^3$ étant tous deux le radical méthyle.

| | |
|---|---|
| Rendement 6 % | Réactif récupéré 20 % |
| Point de fusion | 196,5 - 197 °C |
| Poids moléculaire | 347 g |
| Formule brute | $C_{20}H_{21}N_5O$ |

**EXEMPLE III**

On examine les propriétés photochromiques des composés dans les exemples I et II (cas où $R^6$ = H).

La technique utilisée procède par photolyse à éclairs et permet d'enregistrer le spectre d'absorption de la forme ouverte (photomérocyanine) dans la zone 400-650 nm, ainsi que son évolution au cours du temps.

On détermine ainsi :

Ao : densité optique maximum après photolyse,

$K_\Delta$ : constante cinétique de décoloration (exprimée en $s^{-1}$), ainsi que les longueurs d'onde $\lambda$e à l'épaulement et $\lambda$max au maximum d'absorption, exprimées en nanomètres.

<u>1) Mesures effectuées dans le toluène</u>

Les mesures sont effectuées à une température de 25 °C. L'énergie de photolyse sous 6 KV est d'environ 60 joules. Ao est donné pour une concentration de 2,5 x $10^5$ mole/l. On obtient les résultats suivants :

Composé de l'exemple I :

- Ao = O,14, $K_\Delta$ = 0,17 $s^{-1}$.

Composé de l'exemple II :

- Ao = 0,37, $K_\Delta$ = 0,18 $s^{-1}$.

Les données des séries V et VI ci-après sont données à titre de comparaison avec l'état de la technique :

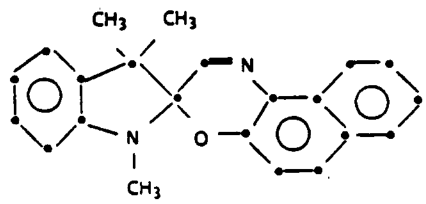

(Série V)

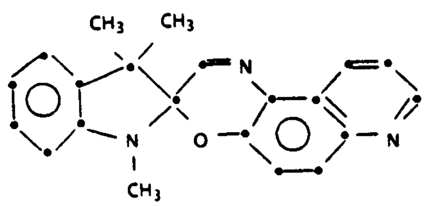

(Série VI)

En choisissant le toluène pour solvant on obtient les longueurs d'onde caractéristiques suivantes extraites du spectre d'absorption :

- Pour la série (V), la longueur d'onde correspondant à l'épaulement de la courbe ($\lambda$e) est de 564 nm, la longueur d'onde maximale ($\lambda$max) correspondant à la valeur de longueur d'onde au maximum de l'absorption est de 594 nm ;
- Pour la série (VI) : $\lambda$e = 561 nm, $\lambda$max = 590 nm
- Pour le composé de l'invention (avec $R^6$ = H), soit :

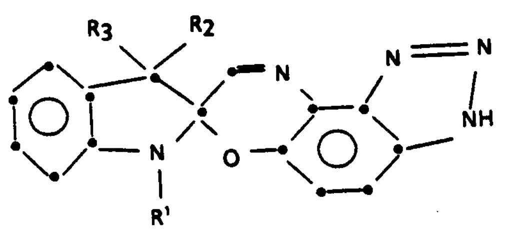

- lorsque $R^1$ = $CH_3$ : $\lambda$e = 595 nm, $\lambda$max = 563 nm
- lorsque $R^1$ = i-$C_3H_7$ : $\lambda$e = 598 nm, $\lambda$max = 563 nm

Pour les composés de l'art antérieur servant de référence, on constate que l'absorption maximum est obtenue pour la plus grande longueur d'onde alors que la situation est inverse pour le composé selon l'invention.

2) Influence du solvant

Les mesures sont effectuées successivement dans le cyclohexane (a), le toluène (b), le diméthyl-sulfoxyde (c).

On prend comme référence un composé connu qui est une spiro(indoline-phénanthrénoxazine) de formule :

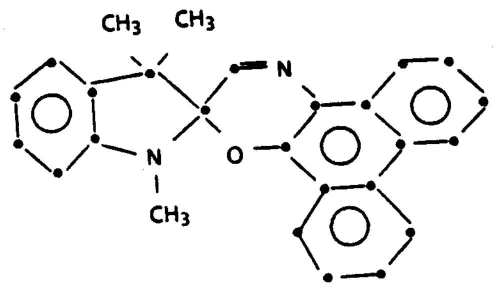

on obtient :

- dans le solvant (a) : λe= 528 nm, λmax = 558 nm
- dans le solvant (b) : λe = 538 nm, λmax = 574 nm
- dans le solvant (c) : λe = 552 nm, λmax = 591 nm

Pour ce composé l'allure du spectre d'absorption de la forme ouverte est la même quel que soit le solvant.

Par contre le composé selon l'invention possède un spectre d'absorption de sa forme ouverte présentant une sensibilité marquée à son environnement, en particulier ici le solvant. Le maximum d'absorption Ao correspond, dans les milieux apolaires aux faibles longueurs d'onde et inversement dans les milieux polaires (en particulier aprotique dipolaire).

Lorsque $R_1$ = i-$C_3H_7$, $R_6$ = H, $R_2$ = $R_3$, $R_4$ = $R_5$ = H (exemple II), on obtient :

- dans le solvant (a) : λe = 595 nm, λmax = 563 nm
- dans le solvant (b) : λe = 598 nm, λmax = 565 nm
- dans le solvant (c) : λe = 569 nm, λmax = 604 nm

On constate que, dans les solvants aprotiques dipolaires, le spectre obtenu après l'éclair de photolyse présente un maximum d'absorption à forte longueur d'onde puis évolue de façon inhabituelle. On observe un phénomène d'interconversion d'une durée de 250 ms dans le diméthylsulfoxyde à 25 °C qui se traduit progressivement par une inversion des bandes d'absorption.

Le nouveau spectre ainsi obtenu (comparable à celui enregistré en milieu apolaire) subit ensuite une décroissance dans le temps correspondant à une décoloration thermique classique.

Un tel phénomène (changement rapide du λmax suite à une excitation lumineuse) peut trouver des applications dans le domaine du stockage d'informations (mémoires d'ordinateur, disques optiques).

**Revendications**

**Revendications pour les Etats contractants suivants : CH, DE, GB, IT, LI**

1. Composés photochromiques de la famille des indolino-spiro-oxazines, caractérisés en ce qu'ils présentent la formule d'un indolinospiro-benzocyclodiazène à groupement benzotriazole, la partie oxazinique de la molécule comportant un cycle triaza orthocondensé sur un cycle aromatique, lesdits composés présentant la formule développée (II) :

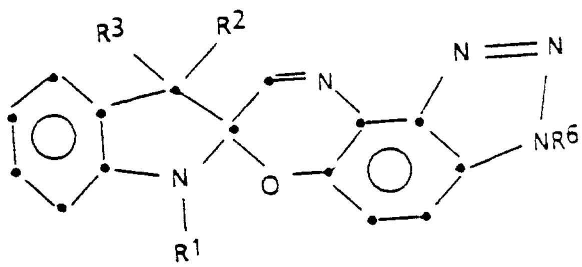

(II)

dans laquelle :

- $R^6$ est un atome d'hydrogène, un phényle, un radical alkyle,
  $R^1$ représente :

  i) un groupe alkyle de 1 à 8 atomes de carbone,
  ii) un groupe allyle, phényle, arylalkyle,
  iii) un groupe alicyclique,
  iv) un groupe hydrocarbure aliphatique comportant dans sa chaîne un ou plusieurs hétéroatomes O, N ou S, une fonction acide, ester ou alcool ;

- $R^2$ et $R^3$ peuvent chacun et indépendamment l'un de l'autre représenter un groupe $C_{1-8}$ alkyle, phényle, phényle mono et disubstitué par des groupes $C_{1-4}$ alkyle et/ou $C_{1-5}$ alkoxy ou peuvent être combinés pour former une chaîne cyclique de 6 à 8 atomes de carbone (incluant le carbone spirannique 3 de l'hétérocycle indolinique) ;
  et qui comporte de 1 à 4 substituants $R^4$ sur le noyau phényle de la partie indolinique de la molécule et/ou 1 ou 2 substituants $R^5$ sur le noyau phényle de la partie oxazinique, $R^4$ et $R^5$ pouvant chacun et indépendamment l'un de l'autre représenter :

  i) un atome d'hydrogène, une fonction amine NR'R", où R' et R" représentent chacun et indépendamment un atome d'hydrogène, un groupe alkyle, cycloalkyle, phényl ; R' et R" peuvent se combiner pour former un cycloalkyle et contenir un ou plusieurs hétéroatomes ;
  ii) un groupe R, OR, SR, COR ou COOR dans lequel R représente un atome d'hydrogène, un groupe alkyle de 1 à 6 atomes de carbone ou un groupe aryle ou hétéroaryle ;
  iii) un atome d'halogène, un groupe $C_{1-4}$ monohaloalkyle, un groupe $C_{1-4}$ polyhaloalkyle,
  iv) $-NO_2$, CN, SCN,

  chacun des substituants $R^4$ pouvant être présent sur l'un quelconque des atomes de carbone convenables de la partie indoline du composé photochromique.

**2.** Composés suivant la revendication 1, caractérisés en ce que le radical $R^6$ est un atome d'hydrogène.

**3.** Composés photochromiques suivant la revendication 1, caractérisés en ce que le substituant $R^6$ est un radical alkyle comportant de 1 à 5 atomes de carbone.

**4.** Composés photochromiques suivant l'une quelconque des revendications précédentes, caractérisés en ce que le substituant $R^1$ est un groupe méthyle, éthyle, n-propyle, isopropyle ou n-butyle.

**5.** Composés photochromiques suivant l'une quelconque des revendications 1 à 3, caractérisés en ce que le substituant $R^1$ est un groupe benzyle, phényle mono et disubstitué par des substituants du type alkyle ou alkoxy de 1 à 6 atomes de carbone.

**6.** Composés photochromiques suivant l'une quelconque des revendications précédentes, caractérisés en ce que $R^2$ et $R^3$ peuvent chacun et indépendamment l'un de l'autre représenter un groupe méthyle ou éthyle.

**7.** Composés photochromiques suivant l'une des revendications 1 à 3, caractérisés en ce que :

- $R^1$ est un groupe $C_{1-4}$ alkyle, phényle ou benzyle ;
- $R^2$ et $R^3$ sont choisis parmi les groupes $C_{1-5}$ alkyle ou un groupe phényle, ou sont combinés pour former un groupe cyclohexyle ;
- chacun des groupes $R^4$ est choisi parmi le groupe constitué par hydrogène, $C_{1-2}$ alkyle, chlore, fluor, brome, iode, $C_{1-2}$ trihaloalkyle et $C_{1-5}$ alkoxy ;
- et $R^5$ est un atome d'hydrogène ou un groupe $C_{1-4}$ alkoxy ou une amine tertiaire ou un halogène.

**8.** Composés photochromiques suivant l'une quelconque des revendications 1 à 7, caractérisés en ce que :

- $R_1$ est un groupe méthyle ou isopropyle ;
- $R_2$ et $R_3$ représentent chacun et indépendamment l'un de l'autre un groupe méthyle ;

**9.** Composés photochromiques suivant l'une quelconque des revendications 7 ou 8, caractérisés en ce que le radical $R^4$ est $-NO_2$ en position 5.

**10.** Compositions photochromiques pour lentilles optiques contenant au moins un des composés suivant l'une quelconque des revendications 1 à 9, en proportion photochromique.

**11.** Procédé de préparation des composés selon l'une quelconque des revendications 1 à 9, caractérisé en ce qu'il comporte une étape de condensation d'une base indolinique III sur un composé nitroso-hydroxyhétéroaromatique IV, suivant le schéma :

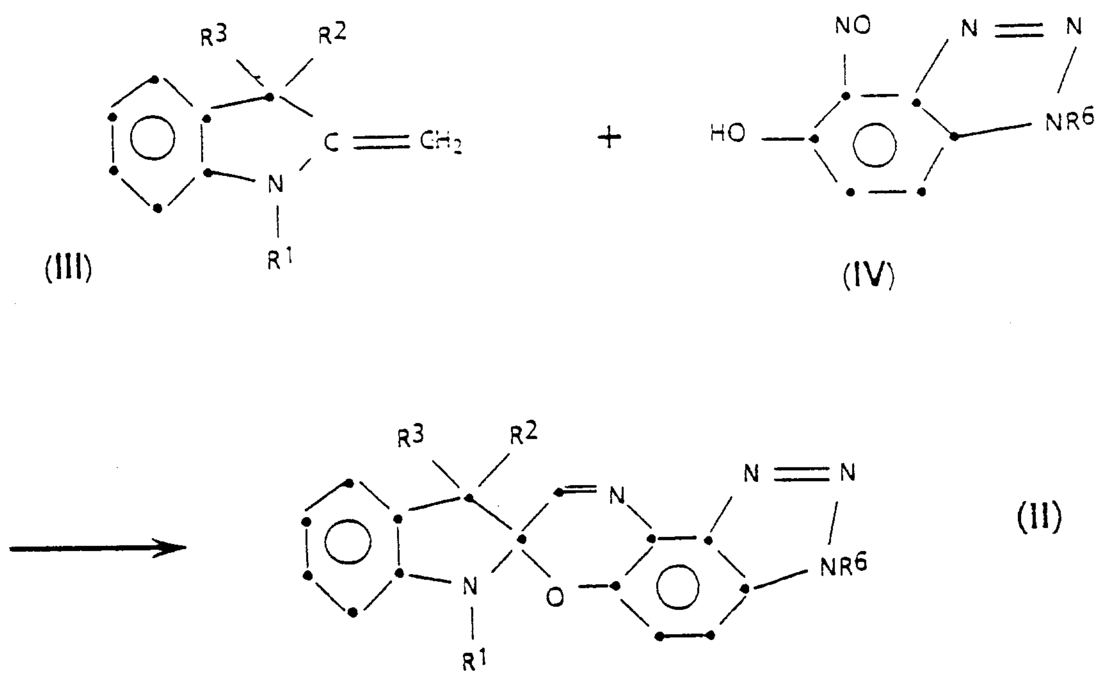

(III) (IV)

(II)

R1, R2, R3, et R6 étant tel que défini dans l'une quelconque des revendications précédentes.

**12.** Procédé suivant la revendication 11, caractérisé en ce que le composé nitroso-hydroxy-hétéroaromatique est l'hydroxy-5 nitroso-4 benzotriazole obtenu par nitrosation d'un dérivé hydroxy résultant de l'hydrolyse d'un dérivé obtenu à partir du para-aminophénol par des étapes successives d'acétylation, nitration, réduction catalytique transformant $NO_2$ en $NH_2$, et condensation pour former le cycle triazolique.

**Revendications pour l'Etat contractant suivant : ES**

**1.** Procédé de préparation de composés photochromique de la famille des indolino-spiro-oxazines, caractérisé en ce qu'il comporte une étape de condensation d'une base indolinique sur un composé nitroso-hydroxy-hétéroaromatique, de manière à obtenir des composés présentant la formule d'un indolinospiro-benzo-cyclodiazène à groupement benzotriazole, dans laquelle la partie oxazinique de la molécule comporte un cyclotriazaorthocondensé sur un cycle aromatique, suivant la formule développée II :

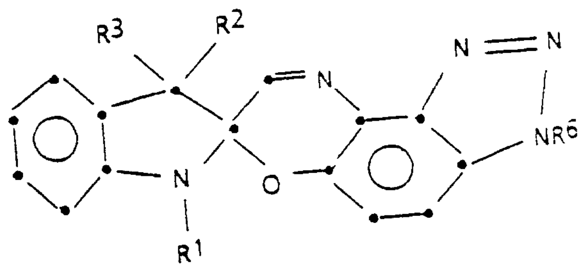

(II)

dans laquelle :

- $R^6$ est un atome d'hydrogène, un phényle, un radical alkyle,
- $R^1$ représente :

  i) un groupe alkyle de 1 à 8 atomes de carbone,
  ii) un groupe allyle, phényle, arylalkyle,
  iii) un groupe alicyclique, un groupe cyclohexyle,
  iv) un groupe hydrocarbure aliphatique comportant dans sa chaîne un ou plusieurs hétéroatomes O, N ou S, une fonction acide, ester ou alcool ;

- $R^2$ et $R^3$ peuvent chacun et indépendamment l'un de l'autre représenter un groupe $C_{1-8}$ alkyle, phényle, phényle mono et disubstitué par des groupes $C_{1-4}$ alkyle et/ou $C_{1-5}$ alkoxy, ou peuvent être combinés pour former une chaîne cyclique de 6 à 8 atomes de carbone (incluant le carbone spirannique 3 de l'hétérocycle indolinique) ;
  et qui comporte de 1 à 4 substituants $R^4$ sur le noyau phényle de la partie indolinique de la molécule et/ou 1 ou 2 substituants $R^5$ sur le noyau phényle de la partie oxazinique, $R^4$ et $R^5$ pouvant chacun et indépendamment l'un de l'autre représenter :

  i) un atome d'hydrogène, une fonction amine NR'R", où R' et R" représentent chacun et indépendamment un atome d'hydrogène, un groupe alkyle, cycloalkyle, phényl ; R' et R" peuvent se combiner pour former un cycloalkyle et contenir un ou plusieurs hétéroatomes ;
  ii) un groupe R, OR, SR, COR ou COOR dans lequel R représente un atome d'hydrogène, un groupe alkyle de 1 à 6 atomes de carbone ou un groupe aryle ou hétéroaryle ;
  iii) un atome d'halogène, un groupe $C_{1-4}$ monohaloalkyle, un groupe $C_{1-4}$ polyhaloalkyle,
  iv) $-NO_2$, CN, SCN,

  chacun des substituants $R^4$ pouvant être présent sur l'un quelconque des atomes de carbone convenables de la partie indoline du composé photochromique.

**2.** Procédé suivant la revendication 1, caractérisé en ce que le radical $R^6$ est un atome d'hydrogène.

**3.** Procédé suivant la revendication 1, caractérisé en ce que le substituant $R^6$ est un radical alkyle comportant de 1 à 5 atomes de carbone.

**4.** Procédé suivant l'une quelconque des revendications précédentes, caractérisé en ce que le substituant $R^1$ est un groupe méthyle, éthyle, n-propyle, isopropyle ou n-butyle.

**5.** Procédé suivant l'une quelconque des revendications 1 à 3, caractérisé en ce que le substituant $R^1$ est un groupe benzyle, phényle mono et disubstitué par des substituants du type alkyle ou alkoxy de 1 à 6 atomes de carbone.

**6.** Procédé suivant l'une quelconque des revendications précédentes, caractérisé en ce que $R^2$ et $R^3$ peuvent chacun et indépendamment l'un de l'autre représenter un groupe méthyle ou éthyle.

**7.** Procédé suivant l'une quelconque des revendications 1 à 3, caractérisé en ce que :

- $R^1$ est un groupe $C_{1-4}$ alkyle, phényle ou benzyle ;
- $R^2$ et $R^3$ sont choisis parmi les groupes $C_{1-5}$ alkyle ou un groupe phényle, ou sont combinés pour former un groupe cyclohexyle ;
- chacun des groupes $R^4$ est choisi parmi le groupe constitué par hydrogène, $C_{1-2}$ alkyle, chlore, fluor, brome, iode, $C_{1-2}$ trihaloalkyle et $C_{1-5}$ alkoxy ;
- et $R^5$ est un atome d'hydrogène ou un groupe $C_{1-4}$ alkoxy ou une amine tertiaire ou un halogène.

**8.** Procédé suivant l'une quelconque des revendications 1 à 7, caractérisé en ce que :

- $R_1$ est un groupe méthyle ou isopropyle ;
- $R_2$ et $R_3$ représentent chacun et indépendamment l'un de l'autre un groupe méthyle ;

**9.** Procédé suivant l'une quelconque des revendications 7 ou 8, caractérisé en ce que le radical $R^4$ est $-NO_2$ en position 5.

**10.** Procédé selon l'une quelconque des revendications 1 à 9, caractérisé en ce qu'il comporte une étape de condensation d'une base indolinique III sur un composé nitroso-hydroxy-hétéroaromatique IV, suivant le schéma :

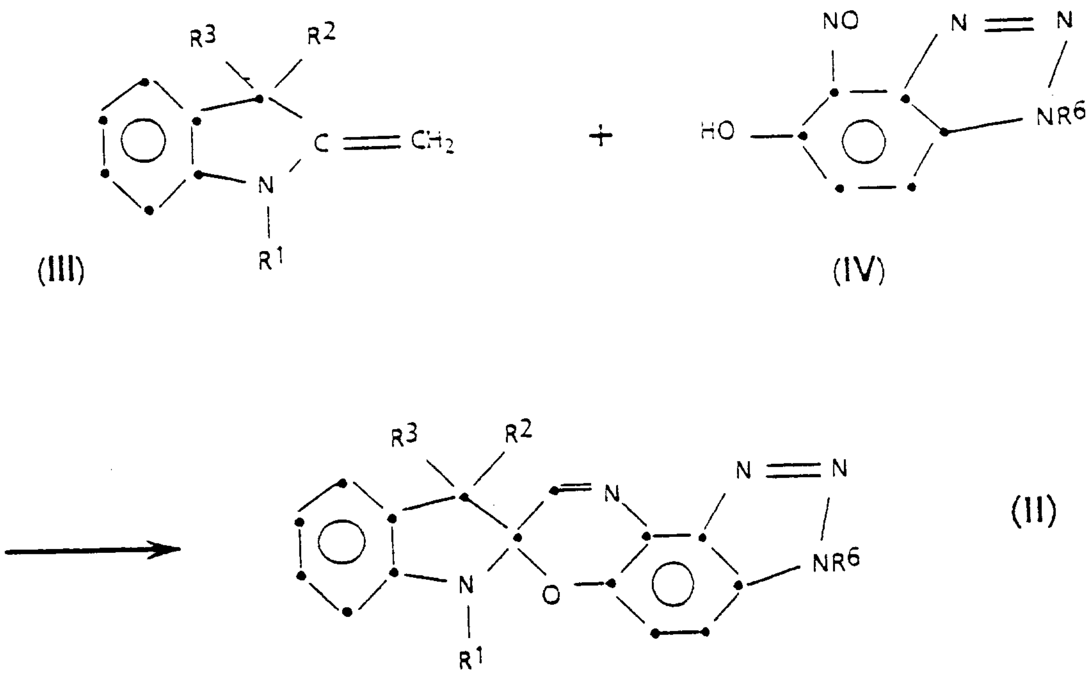

(III) (IV)

(II)

R1, R2, R3, et R6 étant tel que défini dans l'une quelconque des revendications précédentes.

**11.** Procédé suivant la revendication 10, caractérisé en ce que le composé nitroso-hydroxy-hétéroaromatique est l'hydroxy-5 nitroso-4 benzotriazole obtenu par nitrosation d'un dérivé hydroxy résultant de l'hydrolyse d'un dérivé obtenu à partir du para-aminophénol par des étapes successives d'acétylation, nitration, réduction catalytique transformant $NO_2$ en $NH_2$, et condensation pour former le cycle triazolique.

## Claims

### Claims for the following Contracting States : CH, DE, GB, IT, LI

**1.** Photochromic compounds of the family of indolino-spiro-oxazines, characterized in that they possess the formula of an indolino-spiro-benzocyclodiazene with a benzotriazole group, the oxazine portion of the molecule including a triaza ring orthocondensed on an aromatic ring, said compounds having the developed formula (II) :

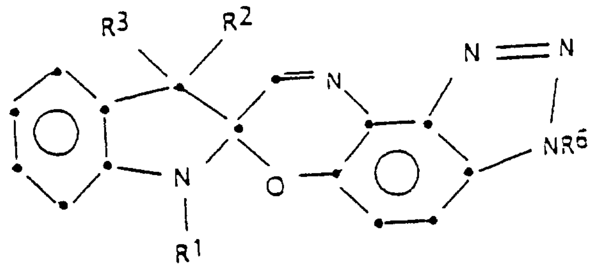

(II)

in which :

- $R^6$ is a hydrogen atom, a phenyl, an alkyl radical,
- $R^1$ represents :

  i) an alkyl group of 1 to 8 carbon atoms,
  ii) an allyl, phenyl, arylalkyl group,
  iii) an alicyclic group,
  iv) an aliphatic hydrocarbon group including in its chain one or several O, N or S heteroatoms, an acid, ester or alcohol function ;

- $R^2$ and $R^3$ can each represent independently of the other a $C_{1-8}$ alkyl group, phenyl, phenyl mono and disubstituted by $C_{1-4}$ alkyl and/or $C_{1-5}$ alkoxy groups or can be combined so as to form a ring chain of 6 to 8 carbon atoms (including the spirane-3 carbon of the indoline heterocycle)
  and which include 1 to 4 substituents $R^4$ on the phenyl nucleus of the indoline portion of the molecule and/or one or two substituents $R^5$ on the phenyl nucleus of the oxazine portion, $R^4$ and $R^5$ being each able to represent independently of the other :

  i) a hydrogen atom, an amine function NR'R", where R' and R" each represent independently a hydrogen atom, an alkyl, cycloalkyl, phenyl group ; R' and R" can combine so as to form a cycloalkyl and contain one or several heteroatoms ;

  ii) an R, OR, SR, COR or COOR group in which R represents a hydrogen atom, an alkyl group of 1 to 6 carbon atoms or an aryl or heteroaryl group ;

  iii) a halogen atom, a $C_{1-4}$ monohaloalkyl group, a $C_{1-4}$ polyhaloalkyl group,

  iv) $-NO_2$, CN, SCN,

  each one of the substituents $R^4$ being able to be present on any one of the suitable carbon atoms of the indoline portion of the photochromic compound.

**2.** Compounds according to claim 1, characterized in that the radical $R^6$ is a hydrogen atom.

**3.** Photochromic compounds according to claim 1, characterized in that the substituent $R^6$ is an alkyl radical including 1 to 5 carbon atoms.

**4.** Photochromic compounds according to any one of the preceding claims, characterized in that the substituent $R^1$ is a methyl, ethyl, n-propyl, isopropyl or n-butyl group.

**5.** Photochromic compounds according to any one of claims 1 to 3, characterized in that the substituent $R^1$ is a benzyl, phenyl mono and disubstituted by the substituents of the type alkyl or alkoxy of 1 to 6 carbon atoms.

**6.** Photochromic compounds according to any one of the preceding claims, characterized in that $R^2$ and $R^3$ can each represent independently of the other a methyl or ethyl group.

7. Photochromic compounds according to one of the claims 1 to 3, characterized in that :

- $R^1$ is a $C_{1-4}$ alkyl, phenyl or benzyl group ;
- $R^2$ and $R^3$ are selected from the $C_{1-5}$ alkyl groups or a phenyl group or are combined so as to form a cyclohexyl group ;
- each group $R^4$ is selected from the group consisting of hydrogen, $C_{1-2}$ alkyl, chlorine, fluorine, bromine, iodine, $C_{1-2}$ trihaloalkyl and $C_{1-5}$ alkoxy ; and
- $R^5$ is a hydrogen atom or a $C_{1-4}$ alkoxy group or a tertiary amine or a halogen.

8. Photochromic compounds according to any one of claims 1 to 7, characterized in that :

- $R^1$ is a methyl or isopropyl group ;
- $R^2$ and $R^3$ each represent independently of the other a methyl group.

9. Photochromic compounds according to any one of claims 7 or 8, characterized in that the $R^4$ radical is $-NO_2$ at the 5 position.

10. Photochromic compositions for optical lenses containing at least one of the compounds according to any one of claims 1 to 9 in a photochromic proportion.

11. Process for the preparation of the compounds of any one of claims 1 to 9, characterized in that it includes a step of condensation of an indoline base III on a nitroso-hydroxy-heteroaromatic compound IV in accordance with the diagram :

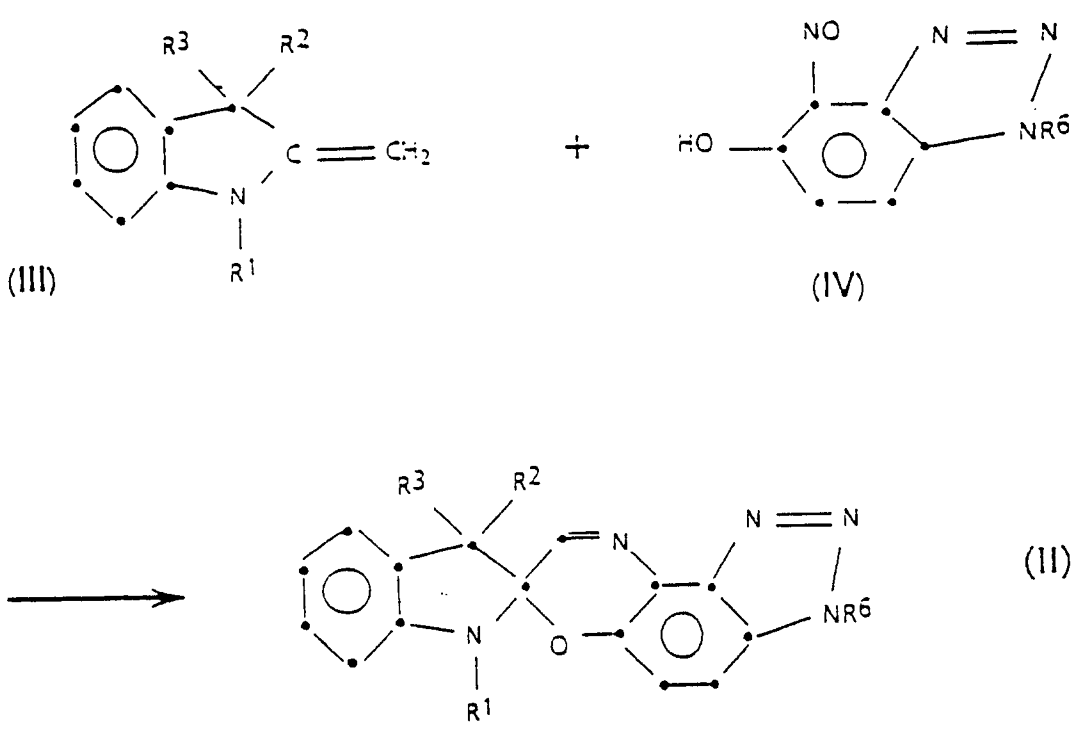
(III) (IV) (II)

$R^1$, $R^2$, $R^3$ and $R^6$ being as defined in any one of the preceding claims.

12. Process according to claim 11, characterized in that the nitroso-hydroxy-heteroaromatic compound is 5-hydroxy 4-nitroso benzotriazole obtained by nitrosation of a hydroxy derivative resulting from the hydrolysis of a derivative

obtained from para-aminophenol by successive steps of acetylation, nitration, catalytic reduction transforming $NO_2$ to $NH_2$, and condensation so as to form the triazole ring.

**Claims for the following Contracting State : ES**

1. Process for the preparation of photochromic compounds of the family of indolino-spiro-oxazines, characterized in that it includes a step of condensation of an indoline base on a nitroso-hydroxy-heteroaromatic compound in a manner so as to obtain the compounds possessing the formula of an indolino-spiro-benzocyclodiazene with a benzotriazole group, in which the oxazine portion of the molecule includes a triaza ring orthocondensed on an aromatic ring, according to the developed formula (II) :

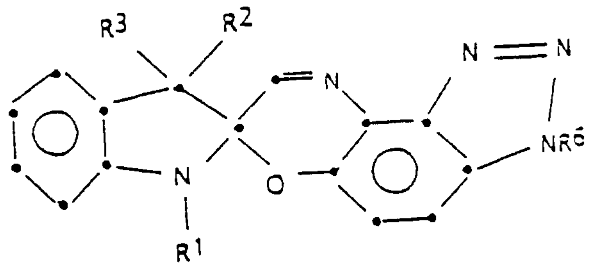

(II)

in which :

- $R^6$ is a hydrogen atom, a phenyl, an alkyl radical,
- $R^1$ represents :

  i) an alkyl group of 1 to 8 carbon atoms,
  ii) an allyl, phenyl, arylalkyl group,
  iii) an alicyclic group, a cyclohexyl group,
  iv) an aliphatic hydrocarbon group including in its chain one or several O, N or S heteroatoms, an acid, ester or alcohol function ;

- $R^2$ and $R^3$ can each represent independently of the other a $C_{1-8}$ alkyl group, phenyl, phenyl mono and disubstituted by $C_{1-4}$ alkyl and/or $C_{1-5}$ alkoxy groups or can be combined so as to form a ring chain containing 6 to 8 carbon atoms (including the spirane-3 carbon of the indoline heterocycle)
  and which include 1 to 4 substituents $R^4$ on the phenyl nucleus of the indoline portion of the molecule and/or one or two substituents $R^5$ on the phenyl nucleus of the oxazine portion, $R^4$ and $R^5$ being each able to represent independently of the other :

  i) a hydrogen atom, an amine function NR'R", where R' and R" each represent independently a hydrogen atom, an alkyl, cycloalkyl, phenyl group ; R' and R" can combine so as to form a cycloalkyl and contain one or several heteroatoms ;

  ii) an R, OR, SR, COR or COOR group in which R represents a hydrogen atom, an alkyl group of 1 to 6 carbon atoms or an aryl or heteroaryl group ;

  iii) a halogen atom, a $C_{1-4}$ monohaloalkyl group, a $C_{1-4}$ polyhaloalkyl group,

  iv) $-NO_2$, CN, SCN,

  each one of the substituents $R^4$ being able to be present on any one of the suitable carbon atoms of the indoline portion of the photochromic compound.

2. Process according to claim 1, characterized in that the radical $R^6$ is a hydrogen atom.

3. Process according to claim 1, characterized in that the substituent $R^6$ is an alkyl radical including 1 to 5 carbon atoms.

**4.** Process according to any one of the preceding claims, characterized in that the substituent $R^1$ is a methyl, ethyl, n-propyl, isopropyl or n-butyl group.

**5.** Process according to any one of claims 1 to 3, characterized in that the substituent $R^1$ is a benzyl, phenyl, mono and disubstituted by the substituents of the type alkyl or alkoxy of 1 to 6 carbon atoms.

**6.** Proces according to any one of the preceding claims, characterized in that $R^2$ and $R^3$ can each represent independently of the other a methyl or ethyl group.

**7.** Process according to any one of claims 1 to 3, characterized in that :

- $R^1$ is a $C_{1-4}$ group comprising alkyl, phenyl or benzyl group ;
- $R^2$ and $R^3$ are selected from the $C_{1-5}$ alkyl groups or a phenyl group or are combined so as to form a cyclohexyl group ;
- each group $R^4$ is selected from the group consisting of hydrogen, $C_{1-2}$ alkyl, chlorine, fluorine, bromine, iodine, $C_{1-2}$ trihaloalkyl and $C_{1-5}$ alkoxy ; and
- $R^5$ is a hydrogen atom or a $C_{1-4}$ alkoxy group or a tertiary amine or a halogen.

**8.** Process according to any one of claims 1 to 7, characterized in that :

- $R^1$ is a methyl or isopropyl group ;
- $R^2$ and $R^3$ each represent independently of the other a methyl group.

**9.** Process according to any one of claims 7 or 8, characterized in that the $R^4$ radical is $-NO_2$ at the 5 position.

**10.** Process of any one of claims 1 to 9 characterized in that it includes a step of condensation of an indoline base III on a nitroso-hydroxy-heteroaromatic compound IV in accordance with the diagram :

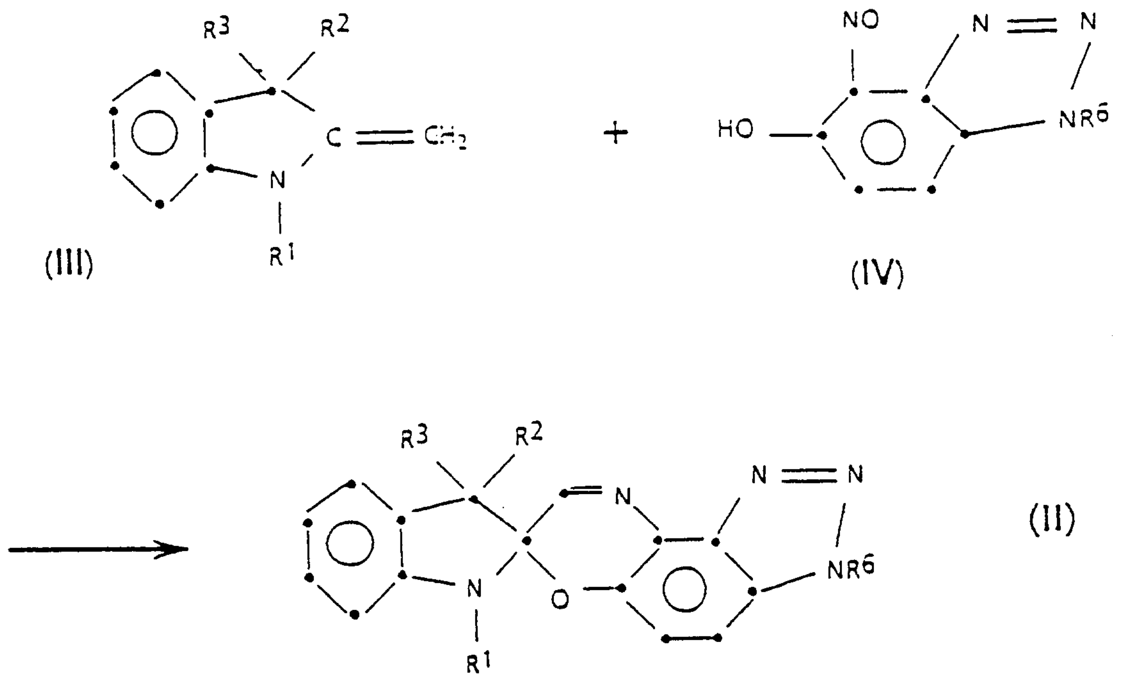

(III) (IV) (II)

$R^1$, $R^2$, $R^3$ and $R^6$ being as defined in any one of the preceding claims.

11. Process according to claim 10, characterized in that the nitroso-hydroxy-heteroaromatic compound is 5-hydroxy 4-nitroso benzotriazole obtained by nitrosation of a hydroxy derivative resulting from the hydrolysis of a derivative obtained from para-aminophenol by successive steps of acetylation, nitration, catalytic reduction transforming $NO_2$ to $NH_2$, and condensation so as to form the triazole ring.

## Patentansprüche

### Patentansprüche für folgende Vertragsstaaten : CH, DE, GB, IT, LI

1. Photochrome Verbindungen aus der Familie der Indolino-spiro-oxazine, dadurch gekennzeichnet, daß sie durch die Formel eines Indolinospiro-benzocyclodiazens an einer Benzotriazolgruppe wiedergegeben werden, wobei der Oxazinanteil des Moleküls einen in ortho-Stellung an den aromatischen Ring kondensierten Triazacyclus (Ring mit 3 Stickstoffatomen) aufweist, wobei die Verbindungen der angegebenen Formel (II) entsprechen:

(II)

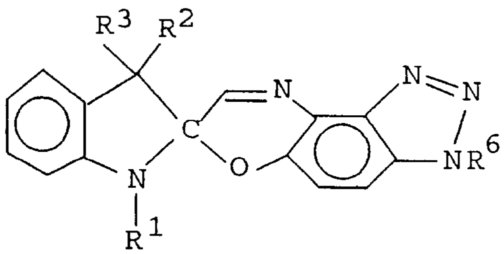

in der

$R^6$ ein Wasserstoffatom, ein Phenylrest, ein Alkylrest ist,
$R^1$

i) eine Alkylgruppe mit 1 bis 8 Kohlenstoffatomen,
ii) eine Allylgruppe, Phenylgruppe, Arylalkylgruppe,
iii) eine alicyclische Gruppe,
iv) eine aliphatische Kohlenwasserstoffgruppe, die in ihrer Kette ein oder mehrere Heteroatome O, N oder S, eine Säurefunktion, Esterfunktion oder Alkoholfunktion aufweist,
bedeutet,

$R^2$ und $R^3$ jeweils und unabhängig voneinander eine $C_1$- bis $C_8$-Alkylgruppe, Phenylgruppe, mit $C_1$- bis $C_4$-Alkyl- und/oder $C_1$-bis $C_5$-Alkoxygruppen mono- oder disubstituierte Phenylgruppe bedeuten können oder kombiniert sein können, um eine cyclische Kette mit 6 bis 8 Kohlenstoffatomen zu bilden (unter Einschluß des Spiro-Kohlenstoffatoms 3 des Indolinheterocyclus),
und die 1 bis 4 Substituenten $R^4$ an dem Phenylkern des Indolinanteils des Moleküls und/oder 1 oder 2 Substituenten $R^5$ an dem Phenylkern des Oxazinanteils umfaßt, wobei $R^4$ und $R^5$ jeweils und unabhängig voneinander

i) ein Wasserstoffatom, eine Aminofunktion NR'R", wobei R' und R" jeweils und unabhängig ein Wasserstoffatom, eine Alkylgruppe, Cycloalkylgruppe, Phenylgruppe bedeuten, wobei R' und R" unter Bildung eines Cycloalkylrestes kombiniert sein können und ein oder mehrere Heteroatome enthalten können,
ii) eine Gruppe R, OR, SR, COR oder COOR, in der R ein Wasserstoffatom, eine Alkylgruppe mit 1 bis 6 Kohlenstoffatomen oder eine Arylgruppe oder Heteroarylgruppe bedeuten,
iii) ein Halogenatom, eine $C_1$- bis $C_4$-Monohalogenalkylgruppe, eine $C_1$- bis $C_4$-Polyhalogenalkylgruppe,
iv) $-NO_2$, CN, SCN

bedeuten können, wobei jeder der Substituenten $R^4$ an jedem geeigneten der Kohlenstoffatome des Indolinanteils der photochromen Verbindung vorhanden sein kann.

2. Verbindungen nach Anspruch 1, dadurch gekennzeichnet, daß der Rest $R^6$ ein Wasserstoffatom ist.

3. Photochrome Verbindungen nach Anspruch 1, dadurch gekennzeichnet, daß der Substituent $R^6$ ein Alkylrest mit 1 bis 5 Kohlenstoffatomen ist.

4. Photochrome Verbindungen nach einem der vorhergehenden Ansprüche, dadurch gekennzeichnet, daß der Substituent $R^1$ eine Methyl-, Ethyl-, n-Propyl-, Isopropyl- oder n-Butylgruppe ist.

5. Photochrome Verbindungen nach einem der Ansprüche 1 bis 3, dadurch gekennzeichnet, daß der Substituent $R^1$ eine Benzylgruppe oder durch Substituenten vom Alkyl- oder Alkoxytyp mit 1 bis 6 Kohlenstoffatomen mono- und disubstituierte Phenylgruppe ist.

6. Photochrome Verbindungen nach einem der vorhergehenden Ansprüche, dadurch gekennzeichnet, daß $R^2$ und $R^3$ jeweils und unabhängig voneinander eine Methylgruppe oder Ethylgruppe bedeuten.

7. Photochrome Verbindungen nach einem der Ansprüche 1 bis 3, dadurch gekennzeichnet, daß

   $R^1$ eine $C_1$- bis $C_4$-Alkylgruppe, Phenylgruppe oder Benzylgruppe ist,
   $R^2$ und $R^3$ ausgewählt sind aus $C_1$- bis $C_5$-Alkylgruppen oder einer Phenylgruppe oder unter Bildung einer Cyclohexylgruppe kombiniert sind,
   jede der Gruppen $R^4$ ausgewählt ist aus der Gruppe bestehend aus Wasserstoff, $C_1$- bis $C_2$-Alkyl, Chlor, Fluor, Brom, Iod, $C_1$- bis $C_2$-Trihalogenalkyl und $C_1$- bis $C_5$-Alkoxy, und
   $R^5$ ein Wasserstoffatom oder eine $C_1$- bis $C_4$-Alkoxygruppe oder ein tertiäres Amin oder ein Halogen ist.

8. Photochrome Verbindungen nach einem der Ansprüche 1 bis 7, dadurch gekennzeichnet, daß

   $R^1$ eine Methylgruppe oder Isopropylgruppe ist,
   $R^2$ und $R^3$ jeweils und unabhängig voneinander eine Methylgruppe bedeuten.

9. Photochrome Verbindungen nach einem der Ansprüche 7 oder 8, dadurch gekennzeichnet, daß der Rest $R^4$ $-NO_2$ in 5-Stellung ist.

10. Photochrome Zusammensetzung für optische Linsen, die mindestens eine der Verbindungen gemäß einem der Ansprüche 1 bis 9 in einem photochromen Anteil enthält.

11. Verfahren zur Herstellung von Verbindungen gemäß einem der Ansprüche 1 bis 9, dadurch gekennzeichnet, daß ein Kondensationsschritt einer Indolinbase III mit einer nitroso-hydroxy-heteroaromatischen Verbindung IV gemäß dem Schema

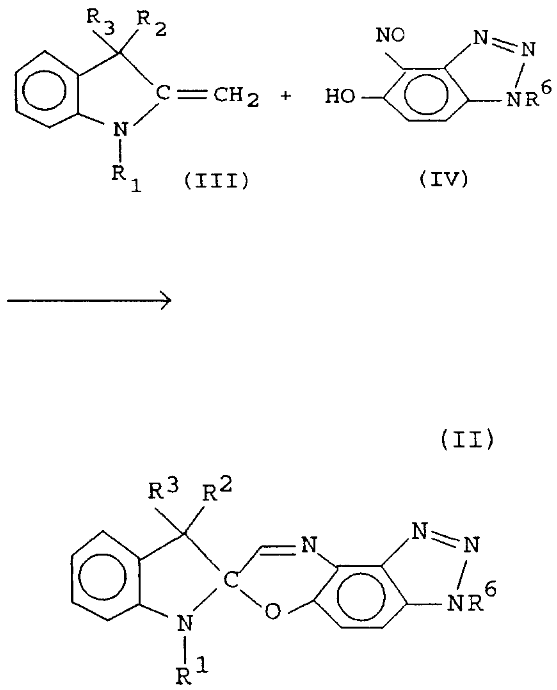

(III) (IV)

(II)

durchgeführt wird, wobei $R^1$, $R^2$, $R^3$ und $R^6$ wie in einem der vorhergehenden Ansprüche definiert sind.

12. Verfahren nach Anspruch 11, dadurch gekennzeichnet, daß die nitroso-hydroxy-heteroaromatische Verbindung 5-Hydroxy-4-nitroso-benzotriazol ist, erhalten durch Nitrosierung eines Hydroxyderivats, das aus der Hydrolyse eines Derivats resultiert, das ausgehend von para-Aminophenol durch aufeinanderfolgende Schritte der Acetylierung, Nitrierung, katalytischen Reduktion, die $NO_2$ in $NH_2$ überführt, und Kondensation zur Bildung des Triazolrings erhalten wird.

**Patentansprüche für folgenden Vertragsstaat : ES**

1. Verfahren zur Herstellung von photochromen Verbindungen aus der Familie der Indolino-spiro-oxazine, dadurch gekennzeichnet, daß ein Kondensationsschritt einer Indolinbase mit einer nitroso-hydroxy-heteroaromatischen Verbindung derart durchgeführt wird, daß Verbindungen mit der Formel eines Indolinospiro-benzocyclodiazens an einer Benzotriazolgruppe erhalten werden, wobei der Oxazinanteil des Moleküls einen in ortho-Stellung an den aromatischen Ring kondensierten Triazacyclus (Ring mit 3 Stickstoffatomen) aufweist, entsprechend der angegebenen Formel (II):

(II)

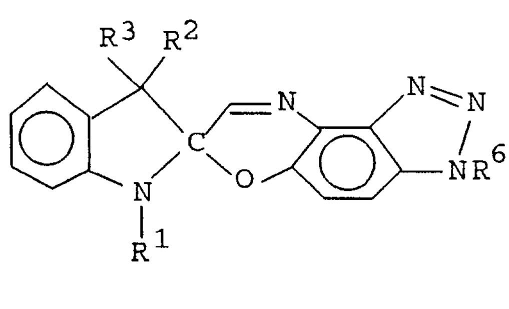

in der

$R^6$ ein Wasserstoffatom, ein Phenylrest, ein Alkylrest ist,

$R^1$

i) eine Alkylgruppe mit 1 bis 8 Kohlenstoffatomen,
ii) eine Allylgruppe, Phenylgruppe, Arylalkylgruppe,
iii) eine alicyclische Gruppe, eine Cyclohexylgruppe,
iv) eine aliphatische Kohlenwasserstoffgruppe, die in ihrer Kette ein oder mehrere Heteroatome O, N oder S, eine Säurefunktion, Esterfunktion oder Alkoholfunktion aufweist,
bedeutet,

$R^2$ und $R^3$ jeweils und unabhängig voneinander eine $C_1$- bis $C_8$-Alkylgruppe, Phenylgruppe, mit $C_1$- bis $C_4$-Alkyl- und/oder $C_1$-bis $C_5$-Alkoxygruppen mono- oder disubstituierte Phenylgruppe bedeuten können oder kombiniert sein können, um eine cyclische Kette mit 6 bis 8 Kohlenstoffatomen zu bilden (unter Einschluß des Spiro-Kohlenstoffatoms 3 des Indolinheterocyclus),
und die 1 bis 4 Substituenten $R^4$ an dem Phenylkern des Indolinanteil des Moleküls und/oder 1 oder 2 Substituenten $R^5$ an dem Phenylkern des Oxazinanteils umfaßt, wobei $R^4$ und $R^5$ jeweils und unabhängig voneinander

i) ein Wasserstoffatom, eine Aminofunktion NR'R", wobei R' und R" jeweils und unabhängig ein Wasserstoffatom, eine Alkylgruppe, Cycloalkylgruppe, Phenylgruppe bedeuten, wobei R' und R" unter Bildung eines Cycloalkylrestes kombiniert sein können und ein oder mehrere Heteroatome enthalten können,
ii) eine Gruppe R, OR, SR, COR oder COOR, in der R ein Wasserstoffatom, eine Alkylgruppe mit 1 bis 6 Kohlenstoffatomen oder eine Arylgruppe oder Heteroarylgruppe bedeuten,
iii) ein Halogenatom, eine $C_1$- bis $C_4$-Monohalogenalkylgruppe, eine $C_1$- bis $C_4$-Polyhalogenalkylgruppe,
iv) $-NO_2$, CN, SCN

bedeuten können, wobei jeder der Substituenten $R^4$ an jedem geeigneten der Kohlenstoffatome des Indolinanteils der photochromen Verbindung vorhanden sein kann.

**2.** Verfahren nach Anspruch 1, dadurch gekennzeichnet, daß der Rest $R^6$ ein Wasserstoffatom ist.

**3.** Verfahren nach Anspruch 1, dadurch gekennzeichnet, daß der Substituent $R^6$ ein Alkylrest mit 1 bis 5 Kohlenstoffatomen ist.

**4.** Verfahren nach einem der vorhergehenden Ansprüche, dadurch gekennzeichnet, daß der Substituent $R^1$ eine Methyl-, Ethyl-, n-Propyl-, Isopropyl- oder n-Butylgruppe ist.

**5.** Verfahren nach einem der Ansprüche 1 bis 3, dadurch gekennzeichnet, daß der Substituent $R^1$ eine Benzylgruppe oder durch Substituenten vom Alkyl- oder Alkoxytyp mit 1 bis 6 Kohlenstoffatomen mono- und disubstituierte Phenylgruppe ist.

**6.** Verfahren nach einem der vorhergehenden Ansprüche, dadurch gekennzeichnet, daß $R^2$ und $R^3$ jeweils und unabhängig voneinander eine Methylgruppe oder Ethylgruppe bedeuten können.

**7.** Verfahren nach einem der Ansprüche 1 bis 3, dadurch gekennzeichnet, daß

$R^1$ eine $C_1$- bis $C_4$-Alkylgruppe, Phenylgruppe oder Benzylgruppe ist,
$R^2$ und $R^3$ ausgewählt sind aus $C_1$- bis $C_5$-Alkylgruppen oder einer Phenylgruppe oder unter Bildung einer Cyclohexylgruppe kombiniert sind,
jede der Gruppen $R^4$ ausgewählt ist aus der Gruppe bestehend aus Wasserstoff, $C_1$- bis $C_2$-Alkyl, Chlor, Fluor, Brom, Iod, $C_1$- bis $C_2$-Trihalogenalkyl und $C_1$- bis $C_5$-Alkoxy, und
$R^5$ ein Wasserstoffatom oder eine $C_1$- bis $C_4$-Alkoxygruppe oder ein tertiäres Amin oder ein Halogen ist.

**8.** Verfahren nach einem der Ansprüche 1 bis 7, dadurch gekennzeichnet, daß

$R^1$ eine Methylgruppe oder Isopropylgruppe ist,
$R^2$ und $R^3$ jeweils und unabhängig voneinander eine Methylgruppe bedeuten.

**9.** Verfahren nach einem der Ansprüche 7 oder 8, dadurch gekennzeichnet, daß der Rest $R^4$ $-NO_2$ in 5-Stellung ist.

**10.** Verfahren nach einem der Ansprüche 1 bis 9, dadurch gekennzeichnet, daß es einen Kondensationsschritt einer Indolinbase III mit einer nitroso-hydroxy-heteroaromatischen Verbindung IV gemäß dem Schema

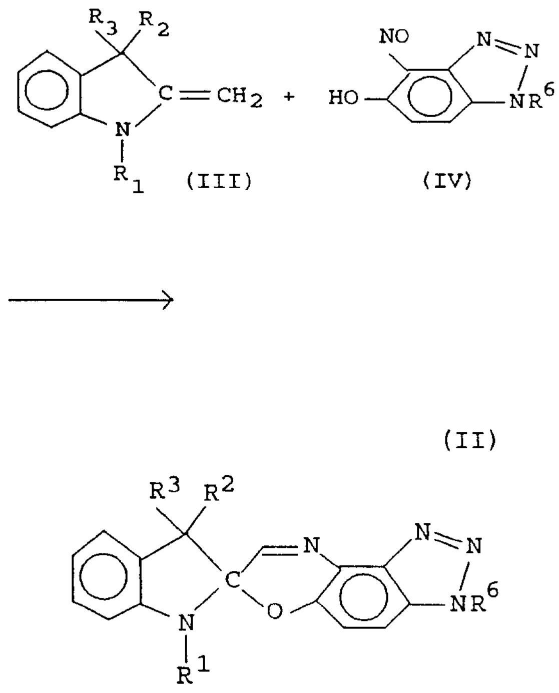

(III) (IV)

(II)

umfaßt, wobei $R^1$, $R^2$, $R^3$ und $R^6$ wie in einem der vorhergehenden Ansprüche definiert sind.

**11.** Verfahren nach Anspruch 10, dadurch gekennzeichnet, daß die nitroso-hydroxy-heteroaromatische Verbindung 5-Hydroxy-4-nitroso-benzotriazol ist, erhalten durch Nitrosierung eines Hydroxyderivats, das aus der Hydrolyse eines Derivats resultiert, das ausgehend von para-Aminophenol durch aufeinanderfolgende Schritte der Acetylierung, Nitrierung, katalytischen Reduktion, die $NO_2$ in $NH_2$ überführt, und Kondensation zur Bildung des Triazolrings erhalten wird.